(19) Europäisches Patentamt
European Patent Office
Office européen des brevets

(11) Publication number: 0 493 683 A1

# (12) EUROPEAN PATENT APPLICATION

(21) Application number: 91120484.0

(22) Date of filing: 29.11.91

(51) Int. Cl.5: A01N 47/12, A01N 47/22, C07C 271/22, C07C 271/54, //(A01N47/12,57:12,47:34,47:14, 47:26,37:46,43:08,43:76), (A01N47/22,57:12,47:34,47:14, 47:26,37:46,43:08,43:76)

(30) Priority: 20.12.90 US 630712

(43) Date of publication of application: 08.07.92 Bulletin 92/28

(84) Designated Contracting States: AT BE CH DE DK ES FR GB GR IT LI LU NL SE

(71) Applicant: AMERICAN CYANAMID COMPANY
1937 West Main Street P.O. Box 60
Stamford Connecticut 06904-0060(US)

(72) Inventor: Hunt, David Allen
9 Water Lily Way
Newtown, PA. 18940(US)

Inventor: Lavanish, Jerome Michael
293 Forrest Road
Yardley, Pennsylvania 19067(US)
Inventor: Asselin, Magda
29 Springwood Drive
Lawrenceville, New Jersey 08648(US)
Inventor: Los, Marinus
107 Drummond Drive
Pennington, New Jersey 08534(US)

(74) Representative: Wächtershäuser, Günter, Dr.
Tal 29
W-8000 München 2(DE)

## (54) Fungicidal amino acid amides.

(57) The present invention relates to a method for the protection of plants from the effects of plant pathogenic fungi by applying to the locus of the plant or the fungi a fungicidally effective amount of a compound having the Formula I structure shown below:

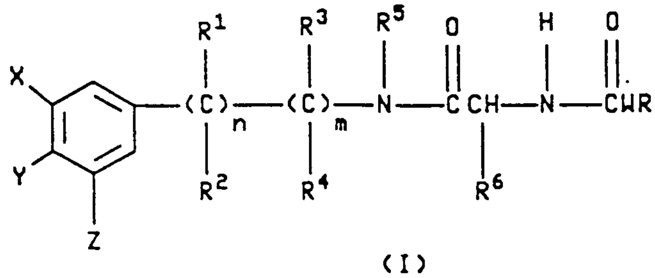

(I)

where X, Y, Z, $R^1$, $R^2$, $R^3$, $R^4$, $R^5$, $R^6$, R n and m have the meanings reported in the description.

Synergistic mixtures with known fungicides such as cymoxanil, fosetyl, mancozeb or metalaxyl are also disclosed.

New compounds of formula I are also disclosed.

EP 0 493 683 A1

## BACKGROUND OF THE INVENTION

Phytopathogenic fungi are the causative agents for many diseases which infect and destroy crops. For example, grape downy mildew, cucumber downy mildew and tomato late blight. To combat this problem a variety of natural and synthetic fungicides have been utilized with varying degrees of success. For example EP 0398072 and EP 0425925 disclose certain fungicidal agents. However even, with the wide variety of fungicides available today diseases caused by fungi still abound. Accordingly, there is an ongoing search in the art to create new and more effective fungicides and methods of controlling and/or preventing fungal infestations.

## SUMMARY OF THE INVENTION

The present invention relates to a method of controlling and/or preventing phytopathogenic fungi, the causative agents for diseases infecting agronomic crops, both growing and harvested. It also relates to fungicidal compounds and compositions which control and/or prevent plant pathogenic fungicidal infestation.

More specifically, the present invention relates to a method for the protection of plants from the effects of plant pathogenic fungi by applying to the locus of the plant or the fungi a fungicidally effective amount of a compound having the Formula I structure shown below:

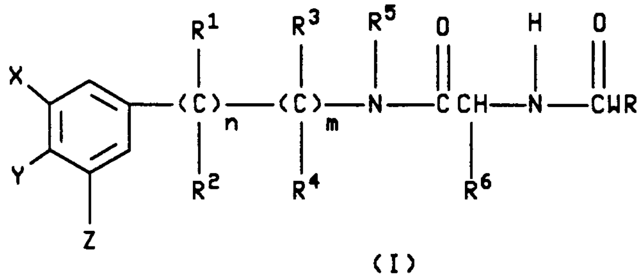

(I)

wherein

| | |
|---|---|
| X, Y and Z | are independently hydrogen, $C_1$-$C_4$ alkyl, $C_1$-$C_4$ alkoxy, $C_3$-$C_4$ alkenyloxy or $C_3$-$C_4$ alkylnyloxy, with the proviso that when n + m = 2, Z is hydrogen, one of X or Y is $C_1$-$C_4$ alkoxy and the other is hydrogen then $R^5$ must be hydrogen; |
| $R^1$, $R^2$, $R^3$, and $R^4$ | are independently hydrogen or $C_1$-$C_4$ alkyl; |
| n and m | are independently integers of 0, 1, 2, or 3 with the proviso that n + m = 1, 2 or 3; |
| $R^5$ | is hydrogen, $C_1$-$C_8$ alkyl, $C_3$-$C_8$ alkenyl, $C_3$-$C_8$ alkynyl, $C_4$-$C_8$ cycloalkylalkyl, $C_7$-$C_{10}$ phenylalkyl, $C_2$-$C_8$ alkoxyalkyl, furylmethyl, thienylmethyl, or imidazolylmethyl; |
| $R^6$ | is 2-propyl, 1-butyl, 2-butyl, 2-methyl-1-propyl, methylthioethyl, or benzyl optionally substituted by one to three $C_1$-$C_4$ alkyl, $C_1$-$C_4$ alkoxy, halo, nitro or cyano; |
| W | is 0 or S; |
| R | is $C_1$-$C_{10}$ alkyl, $C_3$-$C_{10}$ alkenyl or $C_3$-$C_{10}$ alkynyl optionally substituted by from one to three substituents independently selected from<br>$C_1$-$C_4$ alkoxy, $C_1$-$C_4$ alkylthio, halo, nitro, cyano, $R^7$ or $R^8$,<br>or R is $R^7$; |
| $R^7$ | is phenyl, naphthyl, $C_3$-$C_8$ cycloalkyl or $C_3$-$C_8$ cycloalkenyl optionally substituted by from one to three substituents independently selected from<br>$C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, $CHF_2CF_2O$ or $R^8$,<br>or $R^7$ is a 5 or 6 membered heterocyclic ring, containing one or two O or S atoms, optionally substituted by from one to three substituents independently selected from<br>$C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, or $CHF_2CF_2O$; and |
| $R^8$ | is phenoxy, phenylthio, naphthyloxy, naphthylthio,<br>$C_3$-$C_8$ cycloalkyl, $C_3$-$C_8$ cycloalkenyl, $C_3$-$C_8$ cycloalkoxy, $C_3$-$C_8$ cycloalkylthio, $C_3$-$C_8$ cycloalkenyloxy or $C_3$-$C_8$ cycloalkenylthio, optionally substituted by from one |

to three substituents independently selected from

$C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, or $CHF_2CF_2O$.

By the term haloalkyl we intend any compound of the following formula $C_pH_qX_r$ where X is halogen and p, q and r are integers such that $q + r = 2p + 1$.

## Description of the Preferred Embodiments

The antifungal compositions of the present invention include an inert solid or liquid diluent and a compound of having the Formula I structure below:

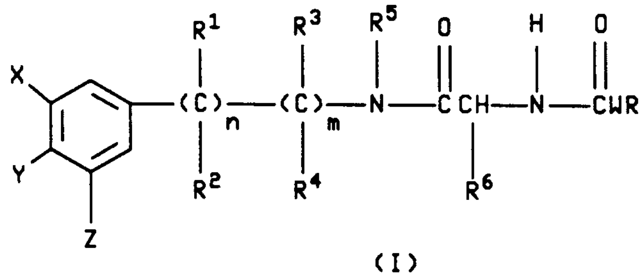

(I)

wherein

| | |
|---|---|
| X, Y and Z | are independently hydrogen, $C_1$-$C_4$ alkyl, $C_1$-$C_4$ alkoxy, $C_3$-$C_4$ alkenyloxy or $C_3$-$C_4$ alkylnyloxy, with the proviso that when $n + m = 2$, Z is hydrogen, one of X or Y is $C_1$-$C_4$ alkoxy and the other is hydrogen then $R^5$ must be hydrogen; |
| $R^1$, $R^2$, $R^3$, and $R^4$ | are independently hydrogen or $C_1$-$C_4$ alkyl; |
| n and m | are independently integers of 0, 1, 2, or 3 with the proviso that $n + m = 1$, 2 or 3; |
| $R^5$ | is hydrogen, $C_1$-$C_8$ alkyl, $C_3$-$C_8$ alkenyl, $C_3$-$C_6$ alkynyl, $C_4$-$C_8$ cycloalkylalkyl, $C_7$-$C_{10}$ phenylalkyl, $C_2$-$C_8$ alkoxyalkyl, furylmethyl, thienylmethyl, or imidazolylmethyl; |
| $R^6$ | is 2-propyl, 1-butyl, 2-butyl, 2-methyl-1-propyl, methylthioethyl, or benzyl optionally substituted by one to three $C_1$-$C_4$ alkyl, $C_1$-$C_4$ alkoxy, halo, nitro or cyano; |
| W | is 0 or S; |
| R | is $C_1$-$C_{10}$ alkyl, $C_3$-$C_{10}$ alkenyl or $C_3$-$C_{10}$ alkynyl optionally substituted by from one to three substituents independently selected from $C_1$-$C_4$ alkoxy, $C_1$-$C_4$ alkylthio, halo, nitro, cyano, $R^7$ or $R^8$, or R is $R^7$; |
| $R^7$ | is phenyl, naphthyl, $C_3$-$C_8$ cycloalkyl or $C_3$-$C_8$ cycloalkenyl optionally substituted by from one to three substituents independently selected from $C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, $CHF_2CF_2O$ or $R^8$, or $R^7$ is a 5 or 6 membered heterocyclic ring, containing one or two O or S atoms, optionally substituted by from one to three substituents independently selected from $C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, or $CHF_2CF_2O$; and |
| $R^8$ | is phenoxy, phenylthio, naphthyloxy, naphthylthio, $C_3$-$C_8$ cycloalkyl, $C_3$-$C_8$ cycloalkenyl, $C_3$-$C_8$ cycloalkoxy, $C_3$-$C_8$ cycloalkylthio, $C_3$-$C_8$ cycloalkenyloxy or $C_3$-$C_8$ cycloalkenylthio, optionally substituted by from one to three substituents independently selected from $C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, or $CHF_2CF_2O$. |

A preferred group of compounds are ones in which the alpha-amino acid portion of the molecule is valine (Formula I, $R^6$ = 2-propyl). Further, preferred are those compounds in which the alpha-amino acid is the L isomer.

Especially preferred are those compounds in which the amino portion of the molecule is a phenethylamino derivative ($n + m = 2$). Most preferred are those compounds in which the phenyl group of

the phenethylamino is 3,4-dimethoxy-phenyl, since these compounds exhibit the highest level of activity against Plasmopera viticola (grape downy mildew) and Phytophthara infestans (tomato late blight).

The present invention also includes a method for controlling and/or preventing phytopathogenic fungi by applying to the locus of the fungi a fungicidally effective amount of a compound having the structure

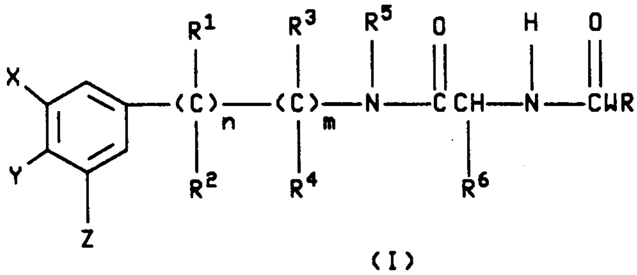

(I)

wherein

| | |
|---|---|
| X, Y and Z | are independently hydrogen, $C_1$-$C_4$ alkyl, $C_1$-$C_4$ alkoxy, $C_3$-$C_4$ alkenyloxy or $C_3$-$C_4$ alkylnyloxy, with the proviso that when n + m = 2, Z is hydrogen, one of X or Y is $C_1$-$C_4$ alkoxy and the other is hydrogen then $R^5$ must be hydrogen; |
| $R^1$, $R^2$, $R^3$, and $R^4$ | are independently hydrogen or $C_1$-$C_4$ alkyl; |
| n and m | are independently integers of 0, 1, 2, or 3 with the proviso that n + m = 1, 2 or 3; |
| $R^5$ | is hydrogen, $C_1$-$C_8$ alkyl, $C_3$-$C_8$ alkenyl, $C_3$-$C_8$ alkynyl, $C_4$-$C_8$ cycloalkylalkyl, $C_7$-$C_{10}$ phenylalkyl, $C_2$-$C_8$ alkoxyalkyl, furylmethyl, thienylmethyl, or imidazolylmethyl; |
| $R^6$ | is 2-propyl, 1-butyl, 2-butyl, 2-methyl-1-propyl, methylthioethyl, or benzyl optionally substituted by one to three $C_1$-$C_4$ alkyl, $C_1$-$C_4$ alkoxy, halo, nitro or cyano; |
| W | is 0 or S; |
| R | is $C_1$-$C_{10}$ alkyl, $C_3$-$C_{10}$ alkenyl or $C_3$-$C_{10}$ alkynyl optionally substituted by from one to three substituents independently selected from $C_1$-$C_4$ alkoxy, $C_1$-$C_4$ alkylthio, halo, nitro, cyano, $R^7$ or $R^8$, or R is $R^7$; |
| $R^7$ | is phenyl, naphthyl, $C_3$-$C_8$ cycloalkyl or $C_3$-$C_8$ cycloalkenyl optionally substituted by from one to three substituents independently selected from $C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, $CHF_2CF_2O$ or $R^8$, or $R^7$ is a 5 or 6 membered heterocyclic ring, containing one or two O or S atoms, optionally substituted by from one to three substituents independently selected from $C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, or $CHF_2CF_2O$; and |
| $R^8$ | is phenoxy, phenylthio, naphthyloxy, naphthylthio, $C_3$-$C_8$ cycloalkyl, $C_3$-$C_8$ cycloalkenyl, $C_3$-$C_8$ cycloalkoxy, $C_3$-$C_8$ cycloalkylthio, $C_3$-$C_8$ cycloalkenyloxy or $C_3$-$C_8$ cycloalkenylthio, optionally substituted by from one to three substituents independently selected from $C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, or $CHF_2CF_2O$. |

Novel compounds useful for the protection of plants from the effects of plant pathogenic fungi have the Formula I structure below:

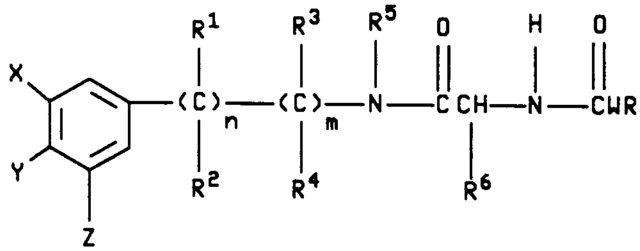

(I)

wherein

X, Y and Z — are independently hydrogen, $C_1$-$C_4$ alkyl, $C_1$-$C_4$ alkoxy, $C_3$-$C_4$ alkenyloxy or $C_3$-$C_4$ alkylnyloxy;

$R^1$, $R^2$, $R^3$, and $R^4$ — are independently hydrogen or $C_1$-$C_4$ alkyl;

n and m — are independently integers of 0, 1, 2, or 3 with the proviso that $n + m = 1$, 2 or 3;

$R^5$ — is hydrogen, $C_1$-$C_8$ alkyl, $C_3$-$C_8$ alkenyl, $C_3$-$C_8$ alkynyl, $C_4$-$C_8$ cycloalkylalkyl, $C_7$-$C_{10}$ phenylalkyl, $C_2$-$C_8$ alkoxyalkyl, furylmethyl, thienylmethyl, or imidazolylmethyl;

$R^6$ — is 2-propyl, 1-butyl, 2-butyl, 2-methyl-1-propyl, methylthioethyl, or benzyl optionally substituted by one to three $C_1$-$C_4$ alkyl, $C_1$-$C_4$ alkoxy, halo, nitro or cyano;

W — is 0 or S;

R — is $C_1$-$C_{10}$ alkyl, $C_3$-$C_{10}$ alkenyl or $C_3$-$C_{10}$ alkynyl optionally substituted by from one to three substituents independently selected from
$C_1$-$C_4$ alkoxy, $C_1$-$C_4$ alkylthio, halo, nitro, cyano, $R^7$ or $R^8$,
or R is $R^7$;

$R^7$ — is phenyl, naphthyl, $C_3$-$C_8$ cycloalkyl or $C_3$-$C_8$ cycloalkenyl optionally substituted by from one to three substituents independently selected from
$C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, $CHF_2CF_2O$ or $R^8$
or $R^7$ is a 5 or 6 membered heterocyclic ring, containing one or two O or S atoms, optionally substituted by from one to three substituents independently selected from
$C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, or $CH_2CF_2O$; and $CHF_2CF_2O$; and

$R^8$ — is phenoxy, phenylthio, naphthyloxy, naphthylthio, $C_3$-$C_8$ cycloalkyl, $C_3$-$C_8$ cycloalkenyl, $C_3$-$C_8$ cycloalkoxy, $C_3$-$C_8$ cycloalkylthio, $C_3$-$C_8$ cycloalkenyloxy or $C_3$-$C_8$ cycloalkenylthio, optionally substituted by from one to three substituents independently selected from
$C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, or $CHF_2CF_2O$.

with the proviso that when $n + m = 2$, Z is hydrogen, one of X or Y is $C_1$-$C_4$ alkoxy and the other is hydrogen, then $R^5$ must be hydrogen;

and with the further proviso that when $n + m = 2$, and $R^1$, $R^2$, $R^3$, $R^4$, $R^5$ and Z are hydrogen (if they exist), X and Y are methoxy, W is 0 and R is t-Butyl or unsubstituted benzyl, then $R^6$ cannot be isobutyl or unsubstituted benzyl;

and with the further proviso that when $n = 1$ and $m = 1$, X and Y are methoxy and Z is hydrogen or methoxy, $R^1$, $R^2$ and $R^4$ are hydrogen, $R^3$ is methyl, R and $R^6$ are unsubstituted benzyl and W is 0, then $R^5$ cannot be hydrogen;

and with the further proviso that when $n + m = 3$, $R^6$ is methylthioethyl or unsubstituted benzyl, W is 0 and R is 1-1-dimethylethyl or 4-methoxyphenylmethyl, then at least one of X or Y must be other than hydrogen;

and with the further proviso that when $n = 1$ and $m = 0$ or $n = 0$ and $m = 1$, $R^1$, $R^3$ and $R^6$ are hydrogen or methyl (if they exist) and W is 0, then R cannot be t-butyl, benzyl or 4-nitrobenzyl.

A preferred group of compounds are those in which $R^6$ is 2-propyl. Also preferred are those compounds in which the alpha amino amide is the L isomer or those compounds in which $n + m = 2$ or 3 (with $n + m = 2$ especially preferred).

The antifungal compounds of the present invention are effective for controlling and/or preventing

phytopathogenic fungi when employed in effective amounts. This will vary somewhat with the virility of the fungus in question and with other factors such as the environment in which treatment is conducted. These compounds are especially useful for the control of fungi which are the causative agents for grape downy mildew and potato and tomato late blight. Certain compounds of the invention may not only be employed to control fungi that has infected the plants, but also may be applied to healthy plants or seeds or to the soil in which the plant is to be grown in order to prevent infestation.

To protect plants from phytopathogenic fungi, the compounds of formula I are applied to the foliage of the plant, to the seed of the plants, or to the soil in which the plant grows or is to be grown, in the form of a liquid, preferably an aqueous spray, or dust, or granular formulation. Solutions or suspensions containing from about 20 ppm to about 1,000 ppm, and preferably 50 ppm to 500 ppm, of formula I compounds are generally effective for this use.

The compounds of the invention may be formulated as emulsifiable concentrates, flowable concentrates, or wettable powders which are diluted with water or other suitable polar solvent, generally in situ, and then applied as a dilute spray. Said compounds may also be formulated in dry compacted granules, granular formulations, dusts, dust concentrates, suspension concentrates, microemulsions and the like all of which lend themselves to seed, soil, water and/or foliage applications to provide the requisite plant protection. Such formulations include the compounds of the invention admixed with inert, pharmacologically acceptable solid or liquid diluents.

For example, wettable powders, dusts, and dust concentrate formulations can be prepared by grinding and blending together about 25% to about 85% by weight of formula I compounds and about 75% to about 15% by weight of a solid diluent such as bentonite, diatomaceous earth, kaolin, attapulgite, or the like, 1% to 5% by weight of a dispersing agent such as sodium lignosulfonate, and about 1% to 5% by weight of a nonionic surfactant, such as octylphenoxy polyethoxy ethanol, nonylphenoxy polyethoxy ethanol or the like.

A typical flowable liquid can be prepared by admixing about 30% to 90% by weight of the active ingredient with about 1% to 3% by weight of a gelling agent such as bentonite, 2% to 5% by weight of a dispersing agent such as sodium lignosulfonate, about 1% by weight of polyethyleneglycol, and about 40% to 60% by weight of water.

A typical emulsifiable concentrate can be prepared by dissolving about 15% to 70% by weight of the active ingredient in about 85% to 30% by weight of a solvent such as isophorone, toluene, butyl cellosolve, methyl acetate, propylene glycol monomethyl ether, or the like and dispersing therein about 1% to 5% by weight of a nonionic surfactant such as an alkylphenoxy polyethoxy alcohol.

Application of the material is made by adding a predetermined quantity of formulated product, such as described above, to the desired volume of water or other suitable solvent, alone or in combination with other agronomic chemicals for simultaneous use.

It is understood that the compounds of the present invention can be applied singly or in combination with one or more other fungicidal compounds, such application being made either by combination of the fungicidal compounds or their formulations in a common container prior to use or by sequential application of the active fungicidal compounds or their formulations to the host crop or its environment. Compounds suitable for combination with the compounds of this invention are suggested by, but not limited to, the following: 4,6-dinitro-o-cresol, benalaxyl, benomyl, captafol, captan, carbendazim, chlorothalonil, copper, cymoxanil, dichlobutrazole, dichlofluanid, diethofencarb, difenconazole, dimethomorph, diniconazole, dinocap, dithianon, fenarimol, fentin acetate, ferbam, flusilazole, folpet, fosetyl, hexaconazole, imazalil, iprodione, mancopper mancozeb, maneb, mepronil, mercuric oxide, metalaxyl, metiram, myclobutanil, nuarimol, ofurace, oxadixyl, penconazole, pencyuron, phosphorous acid, procymidone, propineb, pyrifenox, quintozene, sodium arsenite, sulphur, thiabendazole, thiophanate methyl, thiram, tolclophos-methyl, triadimefon, triadimenol, triforine, vinclozolin, zineb, and/or ziram.

In order to facilitate a further understanding of the invention, the following examples are presented primarily for the purpose of illustrating certain more specific details thereof. The invention is not to be deemed limited thereby except as defined in the claims. Unless otherwise noted, all parts are by weight and all temperatures are expressed as degrees centigrade.

The compounds described herein can be prepared by any of several standard methods known for peptide coupling reactions. The following examples are illustrative of the methods used.

## EXAMPLE 1

### Mixed Anhydride Method

Into a 200mL flask equipped with a paddle stirrer, thermometer and dropping funnel are added 5.00g

(18.85mmol) of L-CBZ-isoleucine, 100mL dry THF, and 1.90g (18.85mmol) N-methylmorpholine. The solution is stirred and cooled to -15°C, at which point 2.46mL isobutyl chloroformate in THF is added dropwise. After the addition, the solution is stirred at -15°C for 15 min., at which point 3.50g (18.85mmol) of 3,4-dimethoxy-B-phenethylamine in THF is added dropwise. The mixture is stirred an additional 1.5 hours, and then the reaction mixture is poured into 500mL water and stirred for half an hour. The slurry is filtered, washed, and dried to give 5.90g (95%) of (D,L)-1-[(3,4-dimethoxyphenethyl)carbamoyl]-n-butyl} carbamic acid benzyl ester. (mp = 117.5 - 118.5°C). Structure confirmed by $^1$HNMR, CIMS, and IR analysis.

In a similar fashion, the Formula II compounds in Table I are prepared.

**TABLE I**

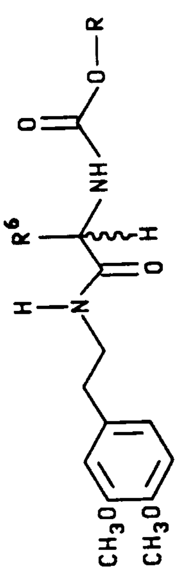

(II)

| Example | mp | $R^6$ | R | Stereochm |
|---|---|---|---|---|
| 2 | 126-28° | 2-propyl | benzyl | D,L |
| 3 | 95-96° | 1-butyl | benzyl | D,L |
| 4 | 87.5-89° | 2-methyl-1-propyl | benzyl | D,L |
| 5 | 145-46° | benzyl | benzyl | L |
| 6 | 122-23° | $CH_2CH_2SCH_3$ | benzyl | L |
| 7 | 144-45.5° | 2-propyl | benzyl | L |

## EXAMPLE 8

## 1,1'-Carbonyldiimidazole-Mediated Coupling

To a THF solution (10mL) of 0.63g (2.38mmol) of 2-carboxyamino-3-methylbutyric acid, N-p-methylbenzyl ester is added 0.39g (2.38mmo1) of 1,1'-carbonyldiimidazole in a single portion. The solution is stirred at ambient temperature for about 15 minutes, at which point 0.46g (2.38mmol) of N-methylhomoveratrylamine in 2mL THF is added in a single portion. The solution is stirred under a $CaCl_2$ drying tube overnight, diluted with $H_2O$ and extracted with $CH_2Cl_2$. The organics are washed with 10% HCl followed by 5% NaOH, dried over $MgSO_4$, filtered, and concentrated to give 0.67g (64%) of 1-[(3,4-dimethoxyphenethyl)-methylcarbamoyl]-2-methylpropylcarbamic acid p- methylbenzyl ester as a clear viscous syrup. Structure is confirmed by $^1$HNMR, CIMS, and IR analyses.

In a similar fashion, the Formula III compounds in Table III are prepared:

**TABLE II**

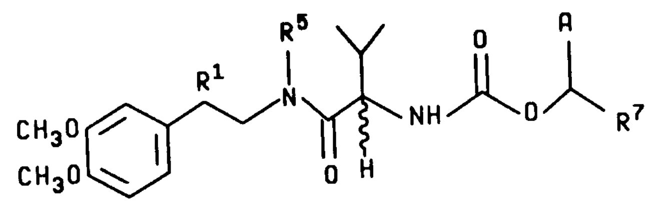

(III)

| Example | mp | $R^1$ | $R^5$ | A | $R^7$ | Stereochem |
|---|---|---|---|---|---|---|
| 9 | syrup | H | $CH_3$ | H | p-$CH_3C_6H_5$ | D,L |
| 10 | syrup | H | $CH_3$ | $CH_3$ | $C_6H_5$ | D,L |
| 11 | 141.5-43° | H | H | H | p-$CH_3C_6H_5$ | D,L |
| 12 | 144-45° | H | H | H | p-$CH_3OC_6H_5$ | D,L |
| 13 | 150-51° | H | H | $CH_3$ | $CH_3$ | D,L |
| 14 | 125-26° | H | $CH_3$ | $CH_3$ | $CH_3$ | D,L |
| 15 | syrup | H | $CH_3$ | H | furyl | D,L |
| 16 | 151.5-54° | H | H | H | furyl | D,L |

**TABLE II (Continued)**

| Example | mp | $R^1$ | $R^5$ | A | $R^7$ | Stereochem |
|---|---|---|---|---|---|---|
| 17 | 104-06° | H | H | H | benzyl | D,L |
| 18 | syrup | H | $CH_3$ | H | benzyl | D,L |
| 19 | 94-95° | H | H | H | $CH_2CH(C_2H_5)C_4H_9$ | D,L |
| 20 | 112-14° | H | H | H | m-$CH_3C_6H_5$ | D,L |
| 21 | 147.5-49° | H | H | H | o-$CH_3C_6H_5$ | D,L |
| 22 | syrup | H | $CH_3$ | H | m-$CH_3C_6H_5$ | D,L |
| 23 | syrup | H | $CH_3$ | H | o-$CH_3C_6H_5$ | D,L |
| 24 | 148-49° | H | H | H | p-$ClC_6H_5$ | D,L |
| 25 | syrup | H | $CH_3$ | H | p-$ClC_6H_5$ | D,L |
| 26 | 118.21° | $CH_3$ | H | H | $C_6H_5$ | L |

TABLE II (Continued)

| Example | mp | $R^1$ | $R^5$ | A | $R^7$ | Stereochem |
|---|---|---|---|---|---|---|
| 27 | 158-59° | H | H | H | $-C\equiv CH$ | D,L |
| 28 | 114-15° | H | H | H | cyclohexyl | D,L |
| 29 | 119-20.5° | H | H | $CH_3$ | $C_2H_5$ | D,L |
| 30 | syrup | H | $CH_3$ | H | cyclohexyl | D,L |
| 31 | syrup | H | $CH_3$ | $CH_3$ | $C_2H_5$ | D,L |
| 32 | gum | H | $CH_3$ | H | 2-naphthyl | D,L |
| 33 | 134-36° | H | H | H | 2-THF# | D,L |
| 34 | syrup | H | $CH_3$ | H | 2-THF# | D,L |
| 35 | 135-36° | H | H | H | $-CH=CH_2$ | D,L |
| 36 | syrup | H | $CH_3$ | H | $-C\equiv CH$ | D,L |

#THF designates tetrahydrofuran

EXAMPLE 37

Method via p-nitrophenylester

To a THF solution (20mL) of 3.72g (10mmol) N-CBZ-L-valine p-nitrophenylester is added a THF solution (5mL) of 3-phenylpropylamine in a single portion. The bright yellow solution is stirred at ambient temperature for 16 hours and is then poured into water and vigorously stirred for 46 minutes. The resulting precipitate is isolated by suction filtration, dissolved in $CH_2Cl_2$, and the solution was washed with saturated $NaHCO_3$ and 5% NaOH, dried over $MgSO_4$, filtered and concentrated to afford 3.08g (84%) of (L)-1-[(3-phenylpropyl)-methylcarbamoyl]-2-methylpropylcarbamic acid benzyl ester as a white powder (mp 131.5 - 133°C). Structure is confirmed by $^1$HNMR, CIMS, and IR analyses.

In a similar fashion, the Formula IV compounds in Table III are prepared:

## TABLE III

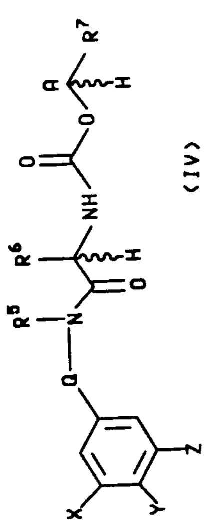

(IV)

| Example | mp | X | Y | Z | $R^5$ | $R^6$ | A | $R^7$ | Q | Stereo |
|---|---|---|---|---|---|---|---|---|---|---|
| 38 | 153-56° | H | H | H | H | 2-propyl | H | Ph | $CH_2CH_2CH(CH_3)$ | D,L;L |
| 39 | syrup | $OCH_3$ | $OCH_3$ | H | $CH_3$ | 2-propyl | H | Ph | $CH_2$ | D,L |
| 40 | gummy | $OCH_3$ | $OCH_3$ | $OCH_3$ | $CH_3$ | 2-propyl | H | Ph | $CH_2CH_2$ | D,L |
| 41 | gummy | $OCH_3$ | $OCH_3$ | H | 2-imidazo-lylmethyl | 2-propyl | H | Ph | $CH_2CH_2$ | D,L |
| 42 | syrup | $OCH_3$ | $OCH_3$ | H | $CH_3$ | isobutyl | H | Ph | $CH_2CH_2$ | L |
| 43 | syrup | $OCH_3$ | $OCH_3$ | H | $CH_3$ | 2-butyl | H | Ph | $CH_2CH_2$ | L |
| 44 | 156-58°C | $OCH_3$ | $OCH_3$ | H | H | 2-propyl | H | Ph | $CH_2CH(CH_3)$ | D,L;D,L |
| 45 | gum | $OCH_3$ | $OCH_3$ | H | $CH_3$ | 2-propyl | H | Ph | $CH_2CH(CH_3)$ | D,L;D,L |
| 46 | gummy | $OCH_3$ | $OCH_3$ | H | i-pr | 2-propyl | H | Ph | $CH_2CH_2$ | D,L |
| 47 | oil | $OCH_3$ | $OCH_3$ | H | et | 2-propyl | H | Ph | $CH_2CH_2$ | L |
| 48 | oil | $OCH_3$ | $OCH_3$ | H | n-pr | 2-propyl | H | Ph | $CH_2CH_2$ | D,L |

### EXAMPLE 49

**Method via N-hydroxysuccinimide Ester**

A THF solution (5mL) of 1.67g (10mmol) veratrylamine is added in one portion to a THF solution 50mL) of 3.48g (10mmol) N-CBZ-D,L-valine-N-hydroxy succinimide ester. The solution is stirred under a $CaCl_2$ drying tube for 25 minutes, diluted with 80mL dimethoxyethane, and stirring continued for 16 hours. The resulting slurry is filtered and the white solid is suction dried to give 0.80g (D,L)-1-[(3,4-dimethoxybenzyl)-carbamoyl]-methylcarbamoyl]-2-methylpropyl carbamic acid benzyl ester as white pellets, mp = 140 - 141.5°C. An additional 1.15g was recovered from the mother liquor (total = 1.95g; 49%). The structure is confirmed by $^1$HNMR, CIMS, and IR analysis.

In a similar fashion, the Formula V compounds in Table IV prepared.

**TABLE IV**

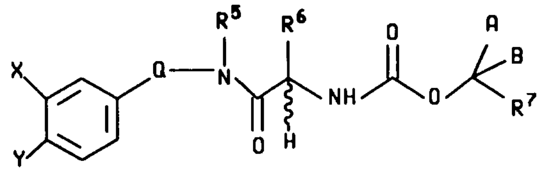

(V)

| Example | mp | X | Y | $R^5$ | $R^6$ | A | B | $R^7$ | Q | Stero |
|---|---|---|---|---|---|---|---|---|---|---|
| 50 | syrup | $OCH_3$ | $OCH_3$ | $CH_2C_6H_5$ | 2-propyl | H | H | Ph | $CH_2CH_2$ | D,L |
| 51 | 154.5-56° | H | H | H | 2-propyl | H | H | Ph | $CH(CH_3)$ | D,L;D,L |
| 52 | syrup | $OCH_3$ | $OCH_3$ | $CH_3$ | benzyl | H | H | Ph | $CH_2CH_2$ | D,L |
| 53 | 105.5-07° | $OCH_3$ | $OCH_3$ | H | 2-propyl | $CH_3$ | $CH_3$ | $CH_3$ | $CH_2CH_2$ | L |
| 54 | syrup | $OCH_3$ | $OCH_3$ | $CH_3$ | 2-propyl | $CH_3$ | $CH_3$ | $CH_3$ | $CH_2CH_2$ | L |
| 55 | syrup | $OCH_3$ | $OCH_3$ | $CH_2C_6H_5$ | 2-propyl | $CH_3$ | $CH_3$ | $CH_3$ | $CH_2CH_2$ | L |
| 56 | syrup | $OCH_3$ | $OCH_3$ | 2-thenyl | 2-propyl | H | H | Ph | $CH_2CH_2$ | D,L |
| 57 | 122° | H | H | H | 2-propyl | $CH_3$ | $CH_3$ | $CH_3$ | $CH(CH_3)$ | D,L;L |
| 58 | 69-73° | $OCH_3$ | $OCH_3$ | $CH_3$ | isobutyl | $CH_3$ | $CH_3$ | $CH_3$ | $CH_2CH_2$ | L |
| 59 | oil | H | H | $CH_3$ | 2-propyl | $CH_3$ | $CH_3$ | $CH_3$ | $CH_2$ | L |

## EXAMPLE 60

**Carbodiimide Method**

To an ice-cooled solution of 1.26g (5.02mmol) N-CBZ-D,L-valine in 7mL $CH_2Cl_2$ is added 1.03g (5.00mmol) 1,3-dicyclohexylcarbodiimide in a single portion. The solution is stirred at 0°C for 10 minutes at which point 1.36g (5.02mmol) N-benzyl-3,4-dimethoxyphenethylamine is added. The reaction mixture is stirred at 0°C for 1 hour, filtered, and the solids washed with $CH_2Cl_2$. The combined filtrate and washings are washed with water, dried, and concentrated. The residue is chromatographed on silica gel, to afford 1.60g (64%) of (D,L)-1-[benzyl(3,4-dimethoxyphenethyl)carbamoyl]-2-methylpropyl carbamic acid benzyl ester as a viscous syrup. Structure confirmed by $^1$HNMR, CIMS and IR analysis.

In a similar fashion, the Formula VI compounds in Table V are prepared.

**TABLE V**

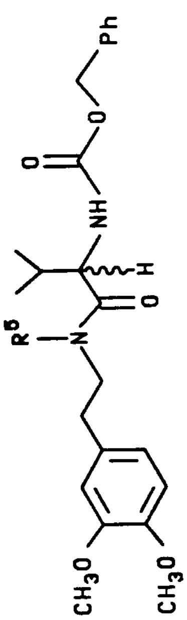

(VI)

| Example | mp | $R^5$ | Stereo |
|---|---|---|---|
| 61 | 116-17° | furfuryl | D,L |
| 62 | gum | $CH_2CH_3$ | D,L |
| 63 | syrup | 2-methyl-1-propyl | D,L |
| 64 | syrup | cyclohexylmethyl | D,L |
| 65 | 99.6-101.6° | $CH(CH_3)C_6H_5$ | D,L |
| 66 | syrup | $CH_3$ | L |
| 67 | syrup | 2-butyl | D,L |
| 68 | oil | 1-butyl | D,L |
| 69 | oil | 1-pentyl | D,L |

## EXAMPLE 70

### Chloroformate Method

To a solution of 2.49g (10mmol) (D,L)-2-amino-N-(3,4-dimethoxyphenethyl)-N,3-dimethylbutyramide and 1.20g (12mmol) triethylamine in 40mL THF is added dropwise at 0°C with stirring 1.87g (11mmol) benzylchloroformate in 10mL THF. After the addition, the reaction mixture is stirred at ambient temperature

for 3 hours. The mixture is concentrated and partitioned between $H_2O$ and $CH_2Cl_2$. The organics are washed with $H_2O$, 3N HCl, $H_2O$, and saturated $NaHCO_3$, dried, filtered, and concentrated. The residue is crystallized/recrystallized to give 2.80g (65%) of (D,L)-1-[(3,4-dimethoxyphenethyl)-methyl-carbomoyl]-2-methylpropyl carbamic acid benzyl ester as a white powder (mp 74 - 76°C). Structure confirmed by $^1$HNMR, CIMS and IR analyses.

In a similar fashion, the Formula VII compounds are prepared and are shown in Table VI.

**TABLE VI**

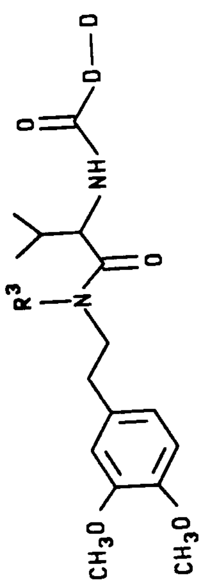

(VII)

| Example | mp | $R^3$ | D | Stereo |
|---|---|---|---|---|
| 71 | 138-40° | H | $CH_2CH_3$ | D,L |
| 72 | 110-11° | $CH_3$ | $CH_2CH_2$ | D,L |
| 73 | glass | $CH_3$ | $C_6H_5$ | D,L |
| 74 | syrup | $CH_3$ | p-$NO_2$-$C_6H_5$ | D,L |
| 75 | syrup | $CH_3$ | p-$NO_2$-$C_6H_5$ | L |
| 76 | syrup | $CH_3$ | $-CH_2-CH(CH_3)_2$ | L |

**EXAMPLES 77-87**

## Additional Fungicidal Compounds

The Formula VIII compounds shown in Table VII are prepared utilizing one of the aforementioned methods.

**TABLE VII**

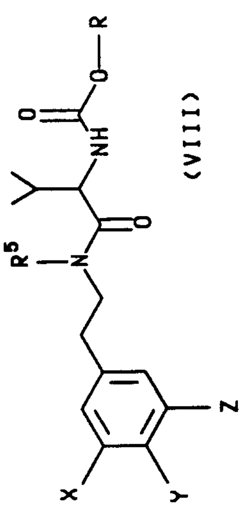

(VIII)

| **Example** | **mp** | **X** | **Y** | **Z** | **$R^5$** | **R** | **Stereochm** |
|---|---|---|---|---|---|---|---|
| 77 | 155.5-57° | $OCH_3$ | $OCH_3$ | H | H | p-$C_6H_4CF_3$ | D,L |
| 78 | Syrup | $OCH_3$ | $OCH_3$ | H | $CH_3$ | p-$C_6H_4CF_3$ | D,L |
| 79 | syrup | $OCH_3$ | $OCH_3$ | H | 2-propyl | benzyl | L |
| 80 | 125-28° | $OCH_3$ | $OCH_3$ | H | H | p-$C_6H_4OCH_3$ | D,L |
| 81 | 154-56° | $OCH_3$ | $OCH_3$ | H | H | p-$C_6H_4Cl$ | D,L |
| 82 | 110-12° | $OCH_3$ | $OCH_3$ | H | H | $CH_2CH_2OCH_3$ | D,L |
| 83 | 105-07° | $OCH_3$ | $OCH_3$ | H | H | $(CH_2)_3Cl$ | D,L |
| 84 | 115.5-18.5° | $OCH_3$ | $OCH_3$ | H | H | $CH_2CH_2OC_6H_5$ | D,L |
| 85 | 136-38° | $OCH_2CH_3$ | H | $OCH_2CH_3$ | H | benzyl | D,L |
| 86 | 151.5-54° | $OCH_3$ | $OCH_3$ | H | H | p-$C_6H_4CH_3$ | D,L |
| 87 | 157-58.5° | $OCH_3$ | $OCH_3$ | H | H | 2-propyl | L |

Example 88

The compounds prepared in examples 1-87 are evaluated as foliar protectants against fungal infection.

Compounds are dissolved in acetone, diluted to the desired concentration with water and surfactant and sprayed onto the test plants. After drying, the test plants are treated with fungal inoculum. When disease symptom development is optimal plants are rated for disease control. Inoculated untreated plants, solvent/surfactant treated plants and plants treated with a reference standard are used for comparison.

Test Organisms

| | |
|---|---|
| Apple scab (AS) | *Venturia inaequalis* |
| Grape downy mildew (GDM) | *Plasmopara viticola* |
| Pepper botritis (PB) | *Botritis cineria* |
| Rice blast (RB) | *Pyricularia oryzae* |
| Sugar beet cercospora (SBC) | *Cercospora beticola* |
| Tomato late blight (TLB) | *Phytophthora infestans* |
| Wheat leaf rust (WLR) | *Puccinia recondita f sp. tritici* |
| Wheat powdery mildew (WPM) | *Erysiphe graminis f sp. tritici* |

Compounds are rated for control of each disease according to the rating scale shown below.

| Rating | % Control |
|---|---|
| 0 | 0 |
| 1 | 1-14 |
| 2 | 15-29 |
| 3 | 39-44 |
| 4 | 45-59 |
| 5 | 60-74 |
| 6 | 75-89 |
| 7 | 90-95 |
| 8 | 96-99 |
| 9 | 100 |

The results are reported in Table VIII.

## TABLE VIII

| (PPM) | 400 | 400 | 100 | 400 | 100 | 400 | 400 | 400 | 400 | 100 | 400 | 400 |
|---|---|---|---|---|---|---|---|---|---|---|---|---|
| Example No. | AS | CDM | CDM | GDM | GDM | PB | RB | SBC | TLB | TLB | WLR | WPM |
| 1 | 4 | 3 | | | | 3 | 0 | 5 | 8 | 7 | 0 | 5 |
| 2 | 0 | 8 | 5 | | | 0 | 0 | 2 | 9 | 9 | 5 | 0 |
| 3 | 2 | 0 | | | | 0 | 3 | 0 | 0 | | 6 | 0 |
| 4 | 0 | 1 | | | | 1 | 1 | 0 | 1 | | 3 | 0 |
| 5 | 0 | 0 | | | | 0 | 1 | 1 | 1 | | 0 | 2 |
| 6 | 2 | 0 | | | | 0 | 4 | 0 | 2 | 1 | 0 | 0 |
| 7 | 0 | 9 | 8 | 9 | 9 | 4 | 0 | 2 | 8 | 9 | 0 | 0 |
| 8 | 0 | | | 9 | 9 | 0 | 0 | 0 | 9 | 8 | 6 | 0 |
| 9 | 0 | | | 9 | 9 | 0 | 0 | 0 | 9 | 9 | 0 | 6 |
| 10 | 0 | | | 6 | | 0 | 0 | 0 | 4 | | 0 | 0 |
| 11 | 0 | | | 9 | 9 | 0 | 0 | 0 | 9 | 9 | 0 | 0 |
| 12 | 0 | | | 9 | 9 | 0 | 0 | 0 | 9 | 9 | 0 | 0 |
| 13 | 7 | | | 9 | 8 | 0 | 0 | 0 | 9 | 9 | 0 | 0 |
| 14 | 0 | | | 9 | 2 | 0 | 0 | 0 | 9 | 9 | 0 | 0 |
| 15 | 0 | | | 9 | 0 | 0 | 0 | 0 | 9 | 8 | 0 | 0 |
| 16 | 0 | | | 9 | 7 | 0 | 6 | 0 | 9 | 9 | 0 | 0 |
| 17 | 0 | | | 9 | 9 | 0 | 0 | 0 | 8 | 8 | 0 | 0 |

**TABLE VIII Continued**

| (PPM) | 400 | 400 | 100 | 400 | 100 | 400 | 400 | 400 | 400 | 100 | 400 | 400 |
|---|---|---|---|---|---|---|---|---|---|---|---|---|
| Example No. | AS | CDM | CDM | GDM | GDM | PB | RB | SBC | TLB | TLB | WLR | WPM |
| 18 | 0 | | | 9 | 9 | 0 | 3 | 0 | 7 | 4 | 0 | 0 |
| 19 | 0 | | | 9 | 9 | 2 | 0 | 4 | 9 | 9 | 0 | 0 |
| 20 | 0 | | | 8 | 9 | 6 | 0 | 0 | 8 | 7 | 0 | 0 |
| 21 | 0 | | | 9 | 8 | 9 | 0 | 0 | 8 | 7 | 0 | 0 |
| 22 | 0 | | | 9 | 7 | 0 | 0 | 0 | 8 | 7 | 6 | 5 |
| 23 | 0 | | | 9 | 4 | 0 | 0 | 0 | 8 | 7 | 6 | 6 |
| 24 | 0 | | | 9 | 9 | 0 | 0 | 0 | 7 | 7 | 0 | 0 |
| 25 | 0 | | | 9 | 7 | 0 | 0 | 0 | 6 | 4 | 7 | 5 |
| 26 | 0 | | | 9 | 0 | 0 | 0 | 0 | 6 | 0 | | |
| 27 | 0 | | | 8 | 8 | 0 | 0 | 0 | 7 | 7 | 0 | 0 |
| 28 | 0 | | | 9 | 8 | 0 | 0 | 0 | 8 | 8 | 0 | 0 |
| 29 | 0 | | | 9 | 9 | 0 | 0 | 0 | 9 | 7 | 0 | 0 |
| 30 | 0 | | | 9 | | 0 | 0 | 0 | 8 | | 0 | 0 |
| 31 | 0 | | | 9 | 7 | 0 | 0 | 0 | 8 | 4 | 0 | 0 |
| 32 | 6 | | | 9 | 7 | 0 | 0 | 0 | 9 | 7 | 6 | 0 |
| 33 | | | | 6 | | 0 | 0 | 0 | 0 | | 0 | 0 |
| 34 | | | | 6 | | 0 | 0 | 0 | 2 | | 0 | 0 |
| 35 | | | | 0 | | 0 | 0 | 0 | 0 | | 6 | 0 |
| 36 | 0 | | | 8 | 6 | 0 | 0 | 0 | 4 | 2 | 0 | 0 |
| 37 | 0 | | | 0 | 0 | 0 | 0 | 6 | 0 | 0 | 0 | 0 |
| 38 | 0 | | | 9 | 7 | 0 | 0 | 0 | 9 | 7 | 0 | 0 |
| 39 | 0 | | | 6 | | 0 | 0 | 0 | 0 | | 0 | 0 |

## TABLE VIII Continued

| (PPM) | 400 | 400 | 100 | 400 | 100 | 400 | 400 | 400 | 400 | 100 | 400 | 400 |
|---|---|---|---|---|---|---|---|---|---|---|---|---|
| Example No. | AS | CDM | CDM | GDM | GDM | PB | RB | SBC | TLB | TLB | WLR | WPM |
| 40 | 0 | | | 6 | 2 | 0 | 0 | 0 | 6 | 0 | 4 | 0 |
| 41 | 0 | | | 4 | | 4 | 0 | 0 | 8 | 7 | 0 | 0 |
| 42 | 0 | | | 7 | 6 | 4 | 0 | 0 | 7 | 7 | 6 | 0 |
| 43 | 0 | | | 9 | 9 | 4 | 0 | 4 | 9 | 9 | 6 | 0 |
| 44 | 0 | | | 8 | 7 | 0 | 0 | 0 | 9 | 9 | 0 | 0 |
| 45 | 4 | | | 9 | 3 | 0 | 0 | 0 | 9 | 8 | 0 | 0 |
| 46 | 0 | | | 9 | 9 | 2 | 0 | 0 | 7 | 6 | 0 | 4 |
| 47 | 0 | | | 9 | 9 | 0 | 0 | 0 | 8 | 7 | 0 | 0 |
| 48 | 0 | | | 9 | 7 | 4 | 0 | 0 | 7 | 4 | 0 | 6 |
| 49 | 0 | | | 0 | | 4 | 0 | 0 | 0 | | 0 | 0 |
| 50 | 0 | | | 4 | 0 | 0 | 0 | 5 | 0 | | 6 | 0 |
| 51 | 0 | | | 3 | 3 | 0 | 4 | 6 | 0 | 0 | 0 | 3 |
| 52 | 0 | | | 3 | 5 | 0 | 4 | 3 | 0 | | 4 | 0 |
| 53 | 0 | | | 9 | 9 | 0 | 0 | 0 | 9 | 9 | 0 | 1 |
| 54 | 0 | | | 9 | 8 | 0 | 0 | 0 | 9 | 7 | 0 | 4 |
| 55 | 0 | | | 6 | 2 | 0 | 0 | 0 | 0 | 0 | 5 | 4 |
| 56 | 0 | | | 7 | 4 | 0 | 8 | 6 | 7 | 4 | 4 | 0 |
| 57 | 0 | | | 9 | 7 | 0 | 0 | 0 | 8 | 4 | 0 | 0 |
| 58 | 0 | | | 0 | | 0 | 0 | 9 | 5 | | 0 | 0 |
| 59 | 0 | | | 0 | 8 | 0 | 0 | 0 | 0 | | 0 | 8 |

## TABLE VIII Continued

| (PPM) | 400 | 400 | 100 | 400 | 100 | 400 | 400 | 400 | 400 | 100 | 400 | 400 |
|---|---|---|---|---|---|---|---|---|---|---|---|---|
| Example No. | AS | CDM | CDM | GDM | GDM | PB | RB | SBC | TLB | TLB | WLR | WPM |
| 60 | 0 | | | 7 | 5 | 0 | 0 | 0 | 0 | | 0 | 0 |
| 61 | 0 | | | 9 | 7 | 0 | 0 | 4 | 0 | | 0 | 0 |
| 62 | 0 | | | 9 | 9 | 1 | 0 | 6 | 9 | 9 | 6 | 1 |
| 63 | 0 | | | 7 | 4 | 0 | 0 | 6 | 0 | | 4 | 3 |
| 64 | 0 | | | 7 | 0 | 0 | 4 | 0 | 0 | | 0 | 0 |
| 65 | 0 | | | 7 | 0 | 0 | 4 | 0 | 0 | | 0 | 0 |
| 66 | 0 | | | 9 | 9 | 0 | 4 | 3 | 9 | 9 | 0 | 0 |
| 67 | 0 | | | 9 | 7 | 0 | 6 | 2 | 6 | | 2 | 4 |
| 68 | 0 | | | 9 | 7 | 0 | 0 | 0 | 7 | 4 | 0 | 0 |
| 69 | 0 | | | 9 | 7 | 0 | 4 | 0 | 0 | 0 | 0 | 0 |
| 70 | 3 | 9 | 8 | 8 | 9 | 4 | 2 | 0 | 9 | 8 | 1 | 2 |
| 71 | 0 | | | 0 | | 0 | 0 | 0 | 9 | 9 | 0 | 0 |
| 72 | 0 | | | 0 | | 0 | 0 | 0 | 9 | 7 | 2 | 5 |
| 73 | 0 | | | 9 | 9 | 0 | 0 | 0 | 9 | 7 | 0 | 0 |
| 74 | 0 | | | 8 | 9 | 0 | 0 | 0 | 8 | 7 | 0 | 4 |
| 75 | 0 | | | 9 | 7 | 2 | 6 | 0 | 9 | 7 | 0 | 0 |
| 76 | 0 | | | 9 | 7 | 0 | 0 | 0 | 9 | 9 | 0 | 0 |
| 77 | 0 | | | 9 | 9 | 0 | 0 | 0 | 0 | 0 | 0 | 0 |

**TABLE VIII Continued**

| (PPM) | 400 | 400 | 100 | 400 | 100 | 400 | 400 | 400 | 400 | 100 | 400 | 400 |
|---|---|---|---|---|---|---|---|---|---|---|---|---|
| Example No. | AS | CDM | CDM | GDM | GDM | PB | RB | SBC | TLB | TLB | WLR | WPM |
| 77 | 0 | | | 8 | 4 | 0 | 0 | 0 | 7 | 0 | 0 | 0 |
| 78 | 0 | | | 8 | 4 | 0 | 0 | 0 | 7 | 0 | 5 | 0 |
| 79 | 0 | | | 7 | 6 | 0 | 0 | 0 | 8 | 7 | 0 | 0 |
| 80 | 5 | | | 9 | 8 | 0 | 0 | 0 | 9 | 9 | 0 | 0 |
| 81 | 0 | | | 9 | 9 | 3 | 0 | 0 | 9 | 9 | 0 | 0 |
| 82 | 0 | | | 4 | 0 | 0 | 0 | 4 | 9 | 9 | 0 | 0 |
| 83 | 0 | | | 6 | 7 | 0 | 0 | 0 | 8 | | 0 | 0 |
| 84 | 0 | | | 7 | 8 | 0 | 0 | 0 | 8 | 8 | 0 | 0 |
| 85 | 0 | | | 9 | 9 | 0 | 0 | 0 | 9 | 8 | 0 | 0 |
| 86 | 0 | | | 9 | | | 0 | 0 | 9 | | 0 | 0 |
| 87 | 6 | | | 3 | | | | 0 | 6 | | | 0 |

## Claims

1. A method for the protection of plants from the effects of plant pathogenic fungi which comprises applying to the locus of the plant or the fungi a synergistic fungicidally effective amount of a composition comprising a first compound selected from the following group: fosetyl, cymoxanil, ferbam mancopper, mancozeb, maneb, metiram, propineb, thiram, zineb, ziram, benalaxyl, metalaxyl, ofurace and oxadixyl and a second compound having the structure shown in Formula I

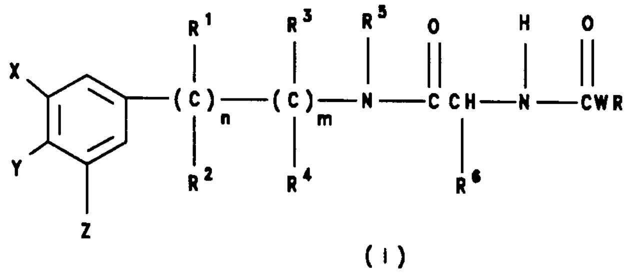

(I)

wherein

X, Y and Z are independently hydrogen, $C_1$-$C_4$ alkyl, $C_1$-$C_4$ alkoxy, $C_3$-$C_4$ alkenyloxy or $C_3$-$C_4$ alkylnyloxy, with the proviso that when n + m = 2, Z is hydrogen, one of X or Y is $C_1$-$C_4$ alkoxy and the other is hydrogen then $R^5$ must be hydrogen;

$R^1$, $R^2$, $R^3$, and $R^4$ are independently hydrogen or $C_1$-$C_4$ alkyl;

n and m are independently integers of 0, 1, 2, or 3 with the proviso that n + m = 1, 2 or 3;

$R^5$ is hydrogen, $C_1$-$C_8$ alkyl, $C_3$-$C_8$ alkenyl, $C_3$-$C_8$ alkynyl, $C_4$-$C_8$ cycloalkylalkyl, $C_7$-$C_{10}$ phenylalkyl, $C_2$-$C_8$ alkoxyalkyl, furylmethyl, thienylmethyl, or imidazolylmethyl;

$R^6$ is 2-propyl, 1-butyl, 2-butyl, 2-methyl-1-propyl, methylthioethyl, or benzyl optionally substituted by one to three $C_1$-$C_4$ alkyl, $C_1$-$C_4$ alkoxy, halo, nitro or cyano;

W is 0 or S;

R is $C_1$-$C_{10}$ alkyl, $C_3$-$C_{10}$ alkenyl or $C_3$-$C_{10}$ alkynyl optionally substituted by from one to three substituents independently selected from $C_1$-$C_4$ alkoxy, $C_1$-$C_4$ alkylthio, halo, nitro, cyano, $R^7$ or $R^8$, or R is $R^7$;

$R^7$ is phenyl, naphthyl, $C_3$-$C_8$ cycloalkyl or $C_3$-$C_8$ cycloalkenyl optionally substituted by from one to three substituents independently selected from $C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, $CHF_2CF_2O$ or $R^8$,
or $R^7$ is a 5 or 6 membered heterocyclic ring, containing one or two O or S atoms, optionally substituted by from one to three substituents independently selected from $C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, or $CHF_2CF_2O$; and

$R^8$ is phenoxy, phenylthio, naphthyloxy, naphthylthio, $C_3$-$C_8$ cycloalkyl, $C_3$-$C_8$ cycloalkenyl, $C_3$-$C_8$ cycloalkoxy, $C_3$-$C_8$ cycloalkylthio, $C_3$-$C_8$ cycloalkenyloxy or $C_3$-$C_8$ cycloalkenylthio, optionally substituted by from one to three substituents independently selected from $C_1$-$C_4$ alkyl, $C_1$-$C_4$ alkoxy, $C_1$-$C_4$ alkylthio, $C_2$-$C_6$ alkoxyalkyl, $C_2$-$C_6$ alkylthioalkyl, cyano, nitro, halo, $C_1$-$C_4$ haloalkyl, $CF_3O$, $CHF_2O$, or $CHF_2CF_2O$.

**2.** The method according to claim 1 wherein the first compound is cymoxanil, fosetyl, mancozeb, or metalaxyl.

**3.** The method according to claim 2 wherein the second compound has structure

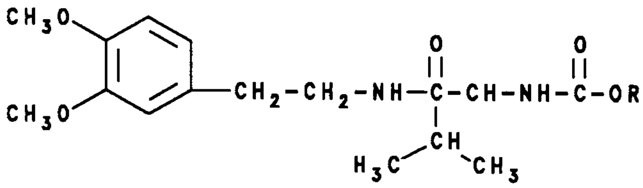

wherein R is tert-butyl, optionally substituted phenyl, or 2-napthyl.

4. The method according to claim 3 wherein the ratio of the first compound to the second compound is about 1:1 to 300:1.

5. The method according to claim 4 wherein the ratio of the first compound to the second compound is about 50:1 to 200:1.

6. The method according to claim 4 wherein the plant pathogenic fungi is the causative agent for grape downy mildew.

7. A compound having the structure

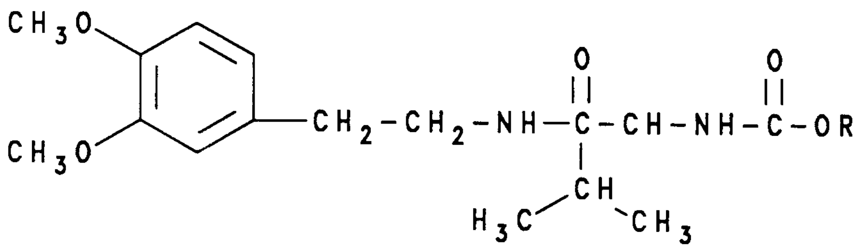

wherein R is tert-butyl, optionally substituted phenyl, or 2-napthyl.

8. The compound according to claim 7 wherein R is tert-butyl, phenyl, 2-napthyl, or parachlorophenyl.

9. The compound according to claim 8 wherein R is tert-butyl or 2-napthyl.

European Patent Office

# EUROPEAN SEARCH REPORT

Application Number

EP 91 12 0484

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,A<br>D | EP-A-0 425 925 (BAYER AG)<br>--- | | A01N47/12<br>A01N47/22<br>C07C271/22<br>C07C271/54<br>//( A01N47/12, 57:12, 47:34, 47:14, 47:26, 37:46, 43:08, 43:76 )<br>( A01N47/22, 57:12, 47:34, 47:14, 47:26, 37:46, 43:08, 43:76 ) |
| D,A | EP-A-0 398 072 (BAYER AG)<br>--- | | |
| A | EP-A-0 257 294 (BAYER AG)<br>--- | | |
| A | EP-A-0 398 059 (BAYER AG)<br>----- | | |

TECHNICAL FIELDS SEARCHED (Int. Cl.5 )

A01N
C07C

The present search report has been drawn up for all claims

| Place of search | Date of completion of the search | Examiner |
|---|---|---|
| THE HAGUE | 30 MARCH 1992 | DONOVAN T.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document